# EUROPEAN PATENT SPECIFICATION

(11) **EP 0 487 645 B1**
(45) Date of publication and mention of the grant of the patent: **13.12.1995**
(21) Application number: 90913995.8
(22) Date of filing: 16.08.1990
(51) Int. Cl.: A61B 17/22, A61B 17/28, A61B 17/32, A61B 10/00, B25B 9/00, B26B 13/26

(54) **A DEVICE FOR GRASPING OR CUTTING AN OBJECT**
ANORDNUNG ZUM GREIFEN ODER SCHNEIDEN EINES GEGENSTANDES
DISPOSITIF POUR SAISIR OU POUR COUPER UN OBJET

(30) Priority: 16.08.1989 US 394463
(43) Date of publication of application: 03.06.1992
(73) Proprietor: RAYCHEM CORPORATION, Menlo Park California 94025 (US)
(72) Inventor: MIDDLEMAN, Lee, M., Portola Valley, CA 94025 (US); PYKA, Walter, R., Redwood City, CA 94061 (US)
(74) Representative: Jay, Anthony William
(86) International application number: US9004624
(87) International publication number: WO9102493

(56) References cited:
- EP-A- 0 069 942
- EP-A- 0 145 166
- US-A- 4 273 128
- US-A- 4 509 517
- US-A- 4 655 219
- PATENT ABSTRACTS OF JAPAN, Volume 11, No. 373 (P-643), 5 December 1987 & JP, A, 62144045 (Toshiba) 5 December 1987 see the Abstract

## Description

This invention relates to a device and to a method of grasping or cutting an object.

Many devices which are used commonly for grasping or cutting objects have two elements which can be moved towards one another and away from one another. The elements have surfaces which may be blunt or sharp so that an object positioned between them may be either grasped or cut when the elements are moved towards one another. Examples of such devices include tongs, tweezers, forceps, scissors, guillotines, and wire cutters.

The elements of such devices are generally rigid, and are moved relative to one another pivotally. The combined requirements that the elements be rigid and capable of pivotal movement can preclude their use in situations where there is a limited amount of space. Furthermore, it can be difficult to manipulate such devices remotely or at angles. These factors tend to make it difficult to use such devices within the body of an animal or human undergoing surgery, especially during less invasive surgical techniques, such as arthroscopy, endoscopy and laproscopy. During such surgery, it may be necessary to grasp and move tissue, for example, to expose an underlying site, or to cut diseased or damaged tissue.

In these less invasive surgical procedures, elongate housings have been developed to position the instrument or device into the body through a minimal incision. Example of such devices are disclosed in U.S. Patents Nos. 3,404,677 to Springer, 3,491,747 to Robinson, 4,218,821 to Schneider, 4,423,729 to Gray, 4,612,708 to Hattori, 4,656,999 to Storz, 4,678,459 to Onik, 4,768,505 to Okada et al, and German Patents Nos. 1,266,446 to Fischer and 3,732,236 to Baumgart.

US-A-4509517 describes an instrument for removing kidney stones. In a typical embodiment the instrument comprises jaws that can move towards or away from each other. Nitinol strips which change their configuration when heated above a critical temperature operate in conjuncation with spring strips exerting a pressure on the Nitinol strips, to cause the Nitinol strips to undergo a shape change when the critical temperature is passed, and thereby cause the jaws to move together or apart. The temperature of the instrument can be changed from below the critical temperature to above the critical temperature in a number of different ways. One way which is simple and quick is to introduce a fluid of the desired temperature through a conduit into the device.

It has now been discovered that one or more elements of such grasping or cutting devices can be formed from a pseudoelastic material, such as a shape memory alloy, which is capable of being greatly deformed without permanent deformation, to provide an improved device that can be more readily used in applications in which there is a limited amount of space. Furthermore, the device can be operated remotely or at angles more conveniently than previously used devices.

It has been proposed to make medical devices from pseudoelastic materials, but such prior art devices typically do not have elements which are splayed apart and then moved toward one another in the manner necessary in grasping or cutting devices, such as scissors. Descriptions of medical devices made from pseudoelastic materials can be found in U.S. Patents Nos. 4,616,656 to Nicholson et al, 4,665,906 to Jervis, 4,898,156 to Gatturna et al, 4.899.743 to Nicholson et al and 4,926,860 to Stice et al, the disclosures of which are incorporated herein by reference.

In one aspect, the present invention provides a device for grasping or cutting an object which comprises
(a) at least two elongate elements positioned alongside one another, each having a body portion and an end portion, the end portions of the elements:
   (i) being capable of being splayed outwardly apart from one another when free of transverse constraint and presenting grasping or cutting surfaces to an object to be grasped or cut that is placed between them; and
   (ii) being capable of being moved inwardly towards one another to grasp or cut said object; and
(b) actuating means
characterised in that a portion of at least one of the elements and/or said actuating means is formed from a material which exhibits pseudoelasticity when deformed under an applied stress.

Another aspect of this invention comprises a device according to the invention which comprises
(a) at least two elongate elements positioned alongside one another, each having a body portion and an end portion the end portions of the elements:
   (i) being capable of being splayed outwardly apart from one another when free of transverse constraint and presenting grasping or cutting surfaces to an object to be grasped or cut that is placed between them; and
   (ii) being capable of being moved inwardly towards one another to grasp or cut said object;
characterised in that the assembly is suitable for use in the device according to the inventon, and in that a portion of at least one of the elements or said actuating means is formed from a material which exhibits pseudoelasticity when deformed under an applied stress.

The pseudoelastic material is preferably a shape memory alloy, such as a nickel/titanium-based alloy.

The device of this invention comprises a hollow elongate component and two elongate elements. Preferably, hollow component is tubular. This has the advantage that the device can be operated remotely.

The material of the hollow component may be polymeric. It may be reinforced, for example, with fibres, to enable it to withstand the forces exerted on it by the elements while they are constrained within and deformed inwardly by the component. A suitable polymeric material for the component is, for example, polytetrafluoroethylene, reinforced with braided fibres. Alternatively, the material of the hollow component may be metallic, for example stainless steel. A preferred hollow component is an elongate tube, preferably formed from stainless steel. The elongate hollow component can be, for example, a tubular housing, cannula, catheter or sheath.

The hollow component may be circular in cross-section which can have the advantage that it permits deformation of the elements substantially uniformly in all directions. Other cross-sections may be preferable in some situations. For example, it can be advantageous to use a hollow component which has the same shape in cross-section as the elements which are received within it, to minimize twisting of the elements relative to one another.

Generally, the elements are at least partially formed from a pseudoelastic material, such as a shape memory alloy, which is capable of being elastically deformed, by as much as 10%, or more depending on the material selected. As discussed above, the device of the invention has the advantage that, by use of elements formed at least partially from a pseudoelastic material,which can be deformed, it can be used in applications in which there is a limited amount of space. Furthermore, the device can be operated remotely or at an angle more conveniently than many previously used devices.

In certain embodiments of the invention, at least one of the end portions of the elements is formed from a pseudoelastic material, preferably a shape memory alloy, and that end portion has a curved configuration when not constrained and is deformed into a straightened configuration when within the hollow component. The term "straightened configuration" means that the configuration of the element is straighter when deformed than it is when the element is not deformed. When the end portion of the element (or end portions of the elements if both are of pseudoelastic material) is extruded from the hollow component it is no longer constrained and reverts or recovers to its curved configuration thereby splaying away from the other element. When the end portion is withdrawn back into the hollow component, or the hollow component is drawn over the end portion, it moves toward the other end portion grasping or cutting any object placed between them.

In some embodiments of the invention, the end portions of the elongate elements are formed from a shape memory alloy and are deformed into a straightened configuration when within the component and curve at an angle to the end of the component when extended therefrom. In certain other embodiments in which the end portions of the elongate elements are formed from a shape memory alloy, they are deformed into a curved configuration when within the component and are substantially straight when extruded from the component. In still other embodiments, the body portion of one or both of the elements is formed from a shape memory alloy and the body portion of the element becomes curved on exiting the component, thereby splaying the end away from the other end portion.

In any embodiment, an actuating means, which may be formed from a shape memory alloy, can be provided to splay the end portions apart from one another and/or to move them toward one another and/or to rotate the elements with respect to the housing and/or slide the elements with respect to the hollow component. In such embodiments, it is not necessary for the elements to be formed from a pseudoelastic material.

At least a portion of at least one, preferably each, of the elements is formed from a shape memory alloy which exhibits pseudoelasticity when deformed under an applied stress. The use of a shape memory which exhibits pseudoelasticity has the advantage that the amount of elastic deformation that is available is large compared with that available from many other materials. In certain preferred embodiments, the end portion of one or both of the elements is formed from a shape memory alloy. In other embodiments, a section of the body portion of one or both of the elements is formed from a shape memory alloy. The large amount of elastic deformation of the elements allows the device to be used to grasp or cut large objects, while ensuring also that the device has a small transverse dimension when the elements are deformed inwardly, allowing the device to pass through small spaces.

The property of pseudoelasticity of certain shape memory alloys, which preferably is used in the device of this invention, is the subject of a paper presented by T. W. Duerig and G. R. Zadno at the International meeting of the Materials Research Society in Tokyo in June 1988. As discussed in the paper, the disclosure of which is incorporated herein by reference, certain alloys are capable of exhibiting pseudoelasticity of two types. "Non-linear pseudoelasticity" arises in appropriately treated alloys while they are in their austenitic phase at a temperature which is greater than Mₛ and less than M_{d} (Mₛ is the temperature at which, when a shape memory alloy in its austenitic phase is cooled, the transformation to the martensitic phase begins, and M_{d} is the maximum temperature at which the transformation to the martensitic phase can be induced by the application of stress). It is generally required that the alloy be annealed at a temperature which is less than the temperature at which the alloy is fully recrystallized. An article formed from an alloy which exhibits non-linear pseudoelasticity can be deformed substantially reversibly by 8% or more. In contrast, "linear pseudoelasticity" is believed not to be accompanied by a phase change. It is exhibited by shape memory alloys which have been cold worked while in the martensitic phase, but have not been annealed in the manner discussed above. An article formed from an alloy which exhibits linear pseudoelasticity can be deformed substantially reversibly by about 4%.

While the alloy that is used in the device of this invention may exhibit either of the extreme types of pseudoelasticity, or pseudoelasticity of an intermediate type, it is generally preferred that it exhibit non-linear pseudoelasticity because of the large amount of elastic deformation that is available, and also because the effective elastic modulus which is characteristic of such deformation is lower, giving rise to the advantages discussed above.

"Non-linear" pseudoelastic properties may be conferred on an article by annealing at a temperature below that at which the alloy is fully recrystallized. The elements may be provided with a desired configuration by holding the arms in that configuration during the annealing step.

A method of treating shape memory alloys to enhance their pseudoelastic properties can be found in U.S. Patent No. 4,935,068 to Duerig, the disclosure of which is incorporated herein by reference.

The material of the elements will be selected according to the characteristics desired of them. For some applications, materials such as spring steel and beryllium copper alloys may be suitable. When a shape memory alloy is used, it is preferably a nickel titanium based alloy, which may include additional elements which might affect the yield strength that is available from the alloy or the temperature at which particular desired pseudoelastic characteristics are obtained. For example, the alloy may be a binary alloy consisting essentially of nickel and titanium, for example 50.8 atomic percent nickel and 49.2 atomic percent titanium, or it may include a quantity of a third element such as vanadium, chromium or iron. Alloys consisting essentially of nickel, titanium and vanadium, such as disclosed in US-4505767, are particularly preferred for some applications, particularly since they can exhibit non-linear pseudoelastic properties at or around body temperatures. Copper based alloys may also be used, for example alloys consisting essentially of copper, aluminum and nickel, copper, aluminum and zinc, and copper and zinc.

The end and body portions of the elongate elements may be formed from the same material, for example, both may be formed from a shape memory alloy, for convenience. Frequently, however, it may be preferable to use different materials because of the different functions that the end and body portions might have to serve. For example, the end portions may be of stainless steel or the like to provide a sharp cutting edge or a cutting edge of stainless steel may be provided on a part of end portions formed from a sharp memory alloy. The cross-sections of the end and body portions will generally be different, although this need not necessarily be the case. For example, the end portions may be rectangular to present a grasping surface or triangular to present a cutting surface, and the body portions may be rectangular for rigidity.

In some embodiments, the end portions of the elements are pivotally connected to one another towards their free ends. This minimizes the possibility of an object to be cut becoming dislocated from the cutting device before it is cut. The device may then be used to move an object once it has been positioned between the elements. This can also be achieved when the elements are not joined together at their free ends, but with less control in some situations. When the elements are not connected directly at their free ends, they may be connected by a flexible component which extends between the end portions of the elements so as, together with the end portions of the elements, to form a closed loop. Leaving the elements unattached at their free ends can facilitate positioning the device so that the object is located between the elements.

The end portions of the elements may be provided with a cutting edge of a material other than a shape memory alloy. The cutting edge may be inlaid in the end portion or can extend from the end portion of the device.

Preferably the body portions of the elements are attached to one another. This can facilitate manipulation of the two elements. For example, the elements may be attached to one another by adhesive material or by fasteners such as screws or rivets, or the elements may be formed as a single body of material. Alternatively, the elements may be attached to an elongate member by which they are moved longitudinally relative to the hollow component. For example, such a member may be hollow, at least at its end, and the elements may be received within the member.

The elongate elements may be symmetrical when they are splayed outwardly apart, and preferably also when deformed inwardly. However, for some applications, it might be appropriate for the elements not to be symmetrical, or for the elements not to be deformed symmetrically (for example only one of the elements might be deformed), or both.

The cutting surfaces of the elements may abut one another in the manner of wire cutters, or they may cross one another in the manner of shears. The grasping surfaces of the elements may abut one another and be sufficiently blunt to avoid cutting the object to be grasped. Alternatively, the grasping surfaces need not be configured so as to contact each other in the manner of cutting devices. The object being grasped need merely be entrapped between the end portions of the elements. The grasping surfaces may be ridged or contain protuberances to assist in grasping the object.

In certain embodiments, an object may be grasped or cut using the device of the invention by bringing together the device and the object while the elements are positioned at least partially within the component, and by then moving the hollow component and the elements longitudinally relative to one another, so that the end portions of the elements extend from the object and become splayed outwardly. The object can then be positioned between the elements to be grasped or cut in accordance with the method described above.

In other embodiments, the end portions of the elements do not splay apart from one another due to pseudoelasticity. In such embodiments, the device is provided with means for actuating the end portions of the elements. Illustrative actuating means are described more fully below with reference to the drawings and include rack and pinion means, pin and slot means, four-bar linkages and the like. In certain embodiments, the actuating means may be pseudoelastic.

The device will be particularly useful in applications in which access to an object to be cut or grasped is restricted, for example in medical applications in which the object to be cut or grasped is a part of a human or animal body. In this application, the elongate elements may be positioned in the body by means of a hollow component in the from of a cannula,catheter or sheath introduced, for example, through an opening into a body cavity.

The device may be arranged so that the axis on which the elements cut the object is not coaxial with the axis of at least a significant portion of the hollow component. This may be arranged, for example, by providing the elements with a suitable bend. The elements may be deformed from their bent configuration towards their straight configuration, and held in the straight configuration, by the hollow component while they are within it. Alternatively, it may be arranged by use of a hollow component which is bent towards the end from which the elements extend.

The device may also be useful in the assembly of mechanical, electrical or other equipment, for example by means of robots.

The invention will be better understood with reference to the accompanying drawings, in which:
Figure 1 is an isometric view of a cutting device of the invention;
Figures 2 (a) to (c) are cross-sections through the device shown in Figure 1, taken at lines A-A, B-B and C-C respectively;
Figures 3 (a) to (e) are elevational views of a first embodiment of the cutting device shown in Figure 1 at various stages during a cutting operation; and
Figures 4 (a) to (c) are elevational views, partially in section, of another embodiment of cutting device at various stages during a cutting operation.
Figures 5 (a) to (e) illustrate an embodiment of a device in accordance with this invention in which the end portions and body portions of the elongate elements are integral and are moved by a rotational actuator made of a material other than a pseudoelastic material.
Figures 6 (a) to (e) illustrate representative cross sections of end portions of the elements adapted to grasp or cut an object.
Figures 7 (a) to (e) illustrate various actuating means which function to cause the elements to splay apart and come together and, optionally, rotate the elements, and/or withdraw the elements into or out of the hollow component.
Figures 8 illustrates an embodiment of the device of this invention in which the end portions are curved when at least partially unconstrained and pinned together pivotally at their tips.
Figure 9 demonstrates a method of using a grasping device of this invention.
Figure 10 (a) to (c) illustrate an embodiment of the device of this invention in which the elements are splayed and in which the body portions of the elements are bent when the elements are unconstrained.
Figure 11(a) and (b) illustrate a device of this invention in which the elements have end portions beyond a pivot point, and in which the body portions of the elements are of pseudoelastic material and when unconstrained are bent to splay the end portions and position them at a desired angle with respect to the hollow component. The body portions act as actuating means to open and close the end portions of the elements to grasp or cut an object.
Figure 12 illustrates a device similar to the device in Figure 11(b) , but in which the body portions of the elements are made of a pseudoelastic material and have a bend of about 90°.

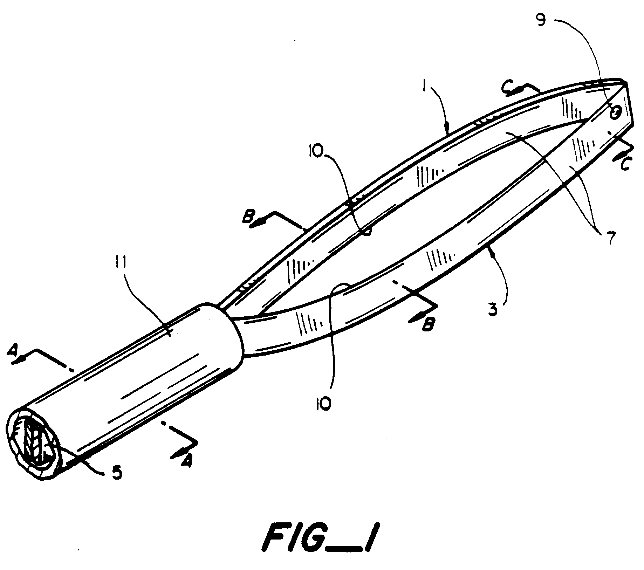

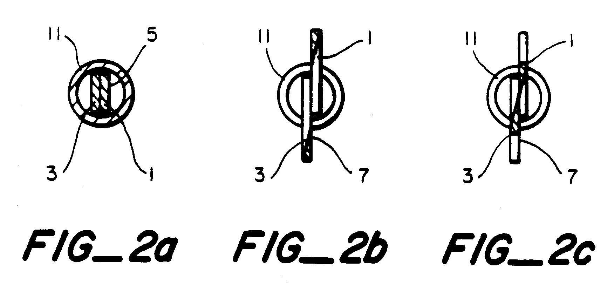

Figures 1 and 2 show a cutting or grasping device which comprises two elongate elements 1 and 3, each having a body portion 5 and an end portion 7. The end portions are joined together pivotally at their free ends by a pin 9. The end portions preferably have a triangular cross-section, where the apex of the triangle provides a cutting surface 10. Alternatively, any flat cross-sectional area may present a grasping surface. Other possible cross-sectional areas are illustrated in Figures 6(a) to (e).

The elongate elements are preferably formed from a shape memory alloy which has been treated so that it exhibits pseudoelasticity in the temperature range between ambient temperature and a temperature above body temperature.

Elongate elements 1 and 3 are located within an elongate housing 11 within which they can slide longitudinally, the housing preferably being a stiff tubular sheath. The elongate elements can be extended beyond the end of housing 11 by longitudinally moving them relative to housing 11 via any suitable manually operated mechanism.

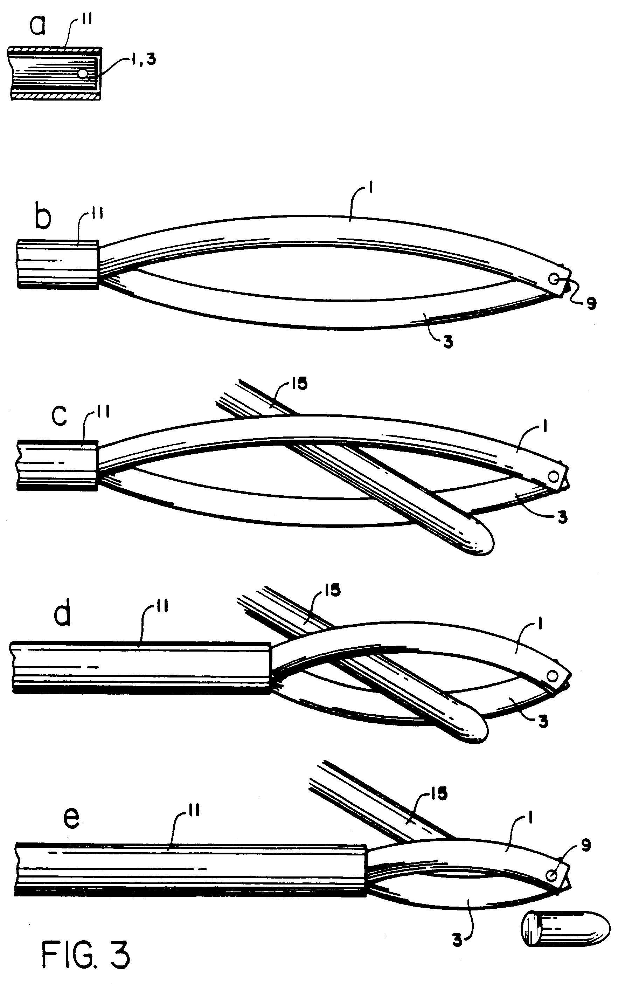

Figure 3 (a) shows the cutting or grasping device with elongate elements 1 and 3 restricted completely within housing 11, which holds the elongate elements in a deformed configuration inwardly towards one another. The housing is positioned as desired relative to an object to be cut while the elongate elements are in this configuration. Once so positioned, the end portions 7 of the elongate elements are caused to extend from the housing, by relative movement of the elements and the housing. Once released from the transverse constraint imposed by the housing, end portions 7 of the elements splay outwardly apart, as shown in Figure 3 (b), allowing an object 15 to be positioned between them, as shown in Figure 3 (c). Figure 2 shows the cross-sectional configurations of elongate elements 1 and 3 at positions A-A, B-B, and C-C of Figure 1, which illustrates the elongate elements splayed apart.

Object 15 is caused to engage the cutting surfaces 10 of elongate elements 1 and 3. Relative longitudinal movement of the elongate elements and the housing will force at least parts of the elongate elements together, thereby cutting the object, as shown in Figures 3 (d) and 3 (e). If desired, object 15 can be moved by holding the housing and moving the elongate elements. If it is desired not to move object 15, the elongate elements are held fixed and the housing is moved. Once object 15 has been cut, the elongate elements are retracted into the housing for removal of the device from the site of the cutting operation.

The end portions 7 of elongate elements 1 and 3 may represent sections of spherical surfaces to facilitate the splaying and cutting. End portions 7 may be used to grasp, instead of cutting, tissues. The grasping function would be facilitated if end portions 7 do not have cutting surfaces 10, and if end portions 7 are not fully retracted into housing 11. Furthermore, the splaying action of elongate elements 1 and 3 may be utilized to separate tissues.

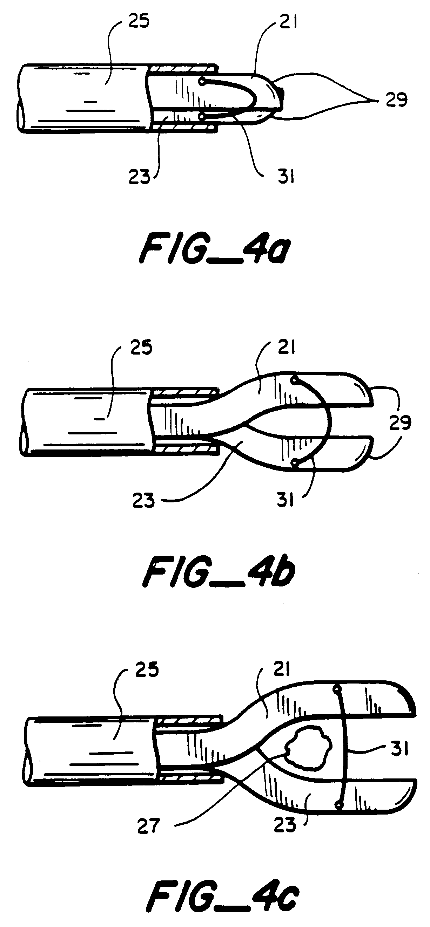

Figure 4 shows a device which comprises two elongate elements 21 and 23 that are preferably formed from a shape memory alloy which has been treated so that it exhibits pseudoelastic behavior. The elements can slide longitudinally within a tubular housing 25. Figure 4 (a) shows the device with the elongate elements 21 and 23 positioned almost entirely within the tubular housing 25. Housing 25 constrains elongate elements 21 and 23 in straightened and deformed shapes.

As elongate elements 21 and 23 are moved longitudinally relative to housing 25, the elongate elements extend from the end of housing 25, as shown in Figures 4 (b) and 4 (c). As they extend from the end of housing 25, the elongate elements become unconstrained and recover toward their preset curved shapes pseudoelastically. They pseudoelastically splay outwardly so that they can receive an object 27 between them. The elongate elements may be interconnected indirectly towards their free ends 29 by a flexible component, such as a piece of wire 31, which helps to prevent dislocation of object 27 from between the elongate elements. Object 27 is cut or grasped by relative movement between housing 25 and the elongate elements, such that the elongate elements become constrained within the housing, generally as described above with reference to Figure 3. The splaying action of elongate elements 21 and 23 may also be utilized to separate tissues.

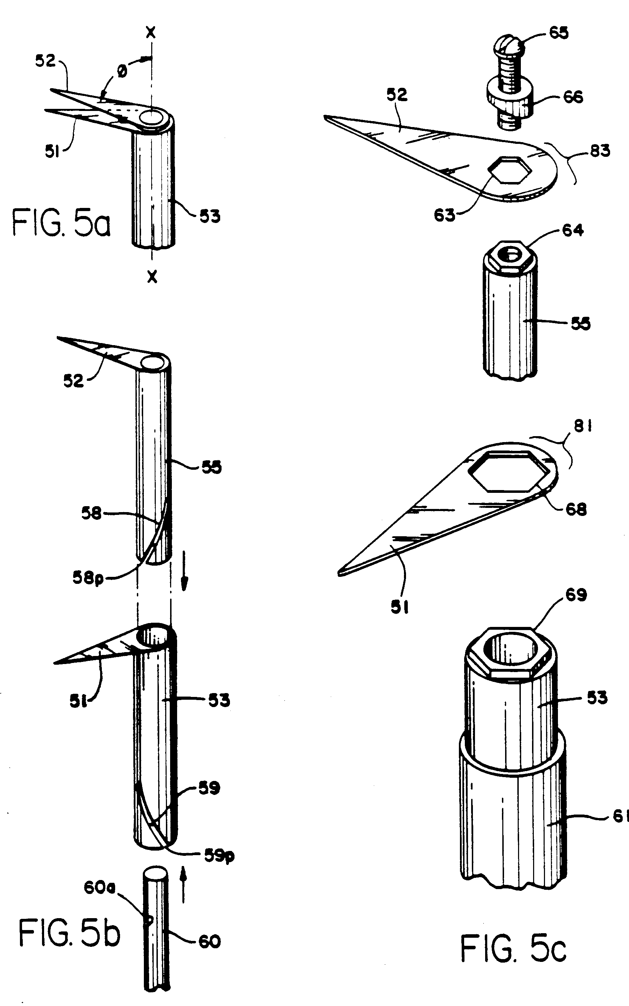

Figure 5 (a) illustrates an embodiment of the invention in which elongate elements 51 and 52 are substantially planar and straight in their unconstrained shapes, but are located in a plane which deviates by an angles φ from a plane which includes the axis x-x of a hollow tube 53. In this embodiment, elongate elements 51 and 52 are attached to outer tube 53 and inner tube 55, respectively, as shown in Figure 5 (b). The proximal end (i.e., the end opposite the elongate element 52) of inner tube 55 is provided with a groove 58, and inner tube 55 is positioned within outer tube 53. The proximal end of outer tube 53 is provided with a groove 59, which extends in a direction opposite to groove 58 of inner tube 55. Plunger 60 is provided with a peg 61. The plunger is positioned at the proximal end of the tubes. The proximal ends 58p and 59p of grooves 58 and 59, respectively, are positioned such that they overlap and are engaged by peg 61. When peg 61 engages proximal ends 58p and 59p of grooves 58 and 59, elongate elements 51 and 52 are preferably splayed apart in the plane defined by their respective flat surfaces. When plunger 60 is pushed into inner tube 55 in a distal direction toward the elongate elements, peg 61 engages grooves 58 and 59, causing tubes 53 and 55, and thereby the elongate elements 51 and 52, to rotate in opposite directions. Preferentially, this rotation would cause the elongate elements to rotate into a more overlapped configuration. The elongate elements can thereby grasp an object placed between them. If the elongate elements have cutting edges, they could thereby cut an object placed between them. When plunger 60 is withdrawn from inner tube 55 again, peg 61 could cause tubes 53 and 55 to rotate such that elongate elements 51 and 52 splay apart from their overlapped configuration. Elongate elements 51 and 52 could thereby be used to separate tissues.

With respect to this embodiment, it should be noted that the angle φ between elongate elements 51 and 52 and tubes 53 and 55 can be any number of degrees desired, even 90 degrees. In addition, the elongate elements may be curved, not only within the plane generally described by their surfaces, but also out of the plane generally described by their surfaces. Furthermore, there may be more than one peg on plunger 60. Correspondingly there would be additional grooves in tubes 53 and 55. The grooves may be spiralled, and longer, such that elongate elements 51 and 52 could be caused to rotate in both directions of their overlapped position in one stroke of plunger 60. Finally, grooves 58 and 59 could be made configured such that elongate elements 51 and 52 could be brought to their overlapped configuration by withdrawing plunger 60 in a proximal direction away from the elongate elements.

Figure 5 (c) shows the attachment of elongate elements 51 and 52 onto inner and outer tubes 53 and 55, respectively. Elongate element 52 is provided with aperture 63 which fits over stem 64, which is integral with or is secured to the distal end of inner tube 55. The length of stem 64 is equal to or less than the thickness of elongate element 52. The cross-sectional shapes of aperture 63 and stem 64 are preferably noncircular, and they may, for example, be square, serrated, notched, etc. Screw 65 and washer 66 fasten elongate element 52 to inner tube 55. Washer 66 may have a beveled side to accommodate the angle φ between the axis x-x of inner tube 55 ( and tube 53) and the plane of elongate elements 51 and 52.

Elongate element 51 is provided with an aperture 68 which fits over stem 69, which is integral with or is secured to the distal end of outer tube 53. The length of stem 69 is preferably slightly greater than the thickness of elongate element 51, so that rotation of elongate element 51 relative to elongate element 52 is not hindered. The cross-sectional shapes of aperture 68 and stem 69 are preferably noncircular, and they may, for example, be square, serrated, notched, etc.

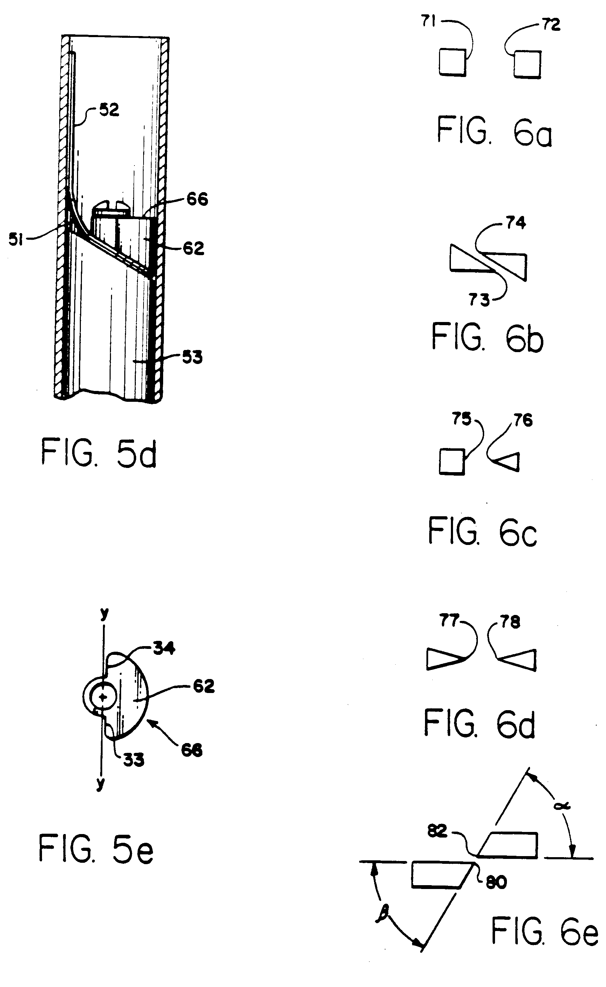

Inner tube 55, with attached elongate element 52, fits into outer tube 53. Elongate element 51 will be captured between the base of stem 69 and elongate element 52. Outer tube 53, with inner tube 55 contained therein, and elongate elements 51 and 52 attached, can be inserted into a sheath 61. As shown in Figure 5 (d), when elongate elements 51 and 52 are drawn into sheath 61 (shown in cross-section), they will be deformed in a direction more parallel to axis x-x. This deformation will be facilitated if elongate elements 51 and 52 are transversely curved along their longitudinal dimensions (i.e., trough shaped). Also, if the outer diameter of tube 53 is only slightly smaller than the inner diameter of sheath 61, the circumferences of elongate elements 51 and 52 along portions 81 and 83 (i.e., the circumferences of elongate elements 51 and 52 around their respective apertures 68 and 63, except for their longitudinally extended portions), should preferably not extend beyond the outer diameter of outer tube 53. When outer tube 53 is extended distally beyond the end of sheath 61, elongate elements 51 and 52 will no longer be constrained, and they will elastically recover their preset shapes again. This deformation and shape recovery is enhanced if the elongate elements are made of a pseudoelastic shape memory alloy.

Figure 5 (e) is a bottom view of a possible embodiment of washer 66. Projection 62 has an outer diameter which is equal to or smaller than the outer diameter of outer tube 53. The surface of projection 62 may be rough, or it may even have teeth or protrusions, in order to obtain a better grip on elongate element 52. Projection 62 preferably encompasses less than half of the circumferential arc of washer 66. The remaining circumference of washer 66 has a outer diameter which is equal to or smaller than the maximum diameter of the head of screw 65. As shown in Figure 5 (d), projection 62 covers the back end 67 of elongate element 52. In this manner, elongate element 51, and secondarily, elongate element 52, can be given as much bending length as possible when they are both constrained within sheath 61. The sides 33 and 34 of projection 62 are preferably perpendicular to the axis y-y of symmetry of washer 66, where axis y-y is perpendicular to the longitudinal dimension of elongate element 52. This will permit ready bending of elongate element 52 along a zone which is perpendicular to its longitudinal dimension.

There may be any suitable means between outer tube 53 and inner tube 55 to prevent plunger 60 from pushing inner tube 55 out of outer 53 tube when plunger 60 is pushed in a distal direction in inner tube 55. In addition, there may be any suitable means between outer tube 53 and sheath 61, so that outer tube 53 can not be completely pushed out of sheath 61 once elongate elements 51 and 52 are adequately extended out of sheath 61 and plunger 60 is used to cause rotation of elongate elements 51 and 52. Plunger 60 can be pushed relative to sheath 61 and tubes 53 and 55 by any suitable manually operated mechanism. Examples of manually operated mechanisms include sliders, pistol grip handles, scissors handles, and syringe-plunger arrangements.

While most of the specific embodiments are directed to cutting devices, it is to be understood that blunt edges can replace the cutting edges in any of the embodiments. Illustrative blunt and cutting edges are shown in Figures 6 (a), (b), (c), (d), and (e). The cutting and grasping edges may be integral with the elements or may be formed separately and attached thereto. Figure 6 (a) illustrates grasping surfaces 71 and 72. Surfaces 71 and 72 may be flat or they may contain ridges, protrusions or the like to aid in gripping an object. Figure 6 (b) illustrates shearing cutting edges 73 and 74 which cut an object by a shearing action. Figure 6 (c) illustrates another pair of edges for cutting. In Figure 6 (c), surface 75 is flat, while edge 76 provides a sharp edge for cutting an object. Figure 6 (d) illustrates cutting edges 77 and 78. Sharp edges 77 and 78 of the triangular cross-sections meet to permit cutting. Figure 6 (e) illustrates cutting edges 80 and 82, which are at any desired angles a and β relative to the direction of opening and closing of the elongate elements. In all of these embodiments, as well as in all of the embodiments described herein, the cutting edges or gripping surfaces could be made of any material such as steel, diamond, plastic, etc., which is attached to the elongate elements.

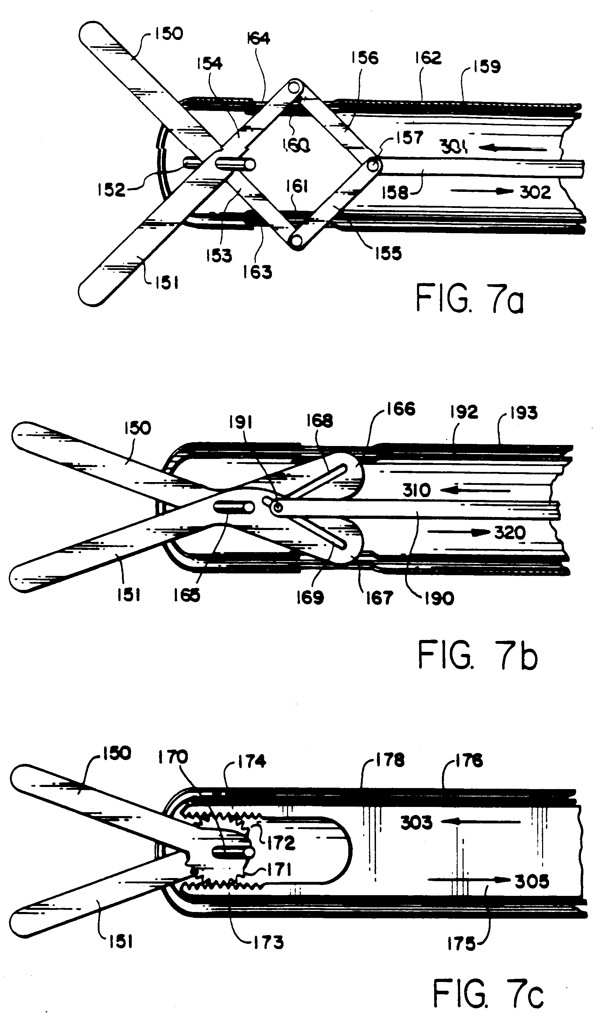

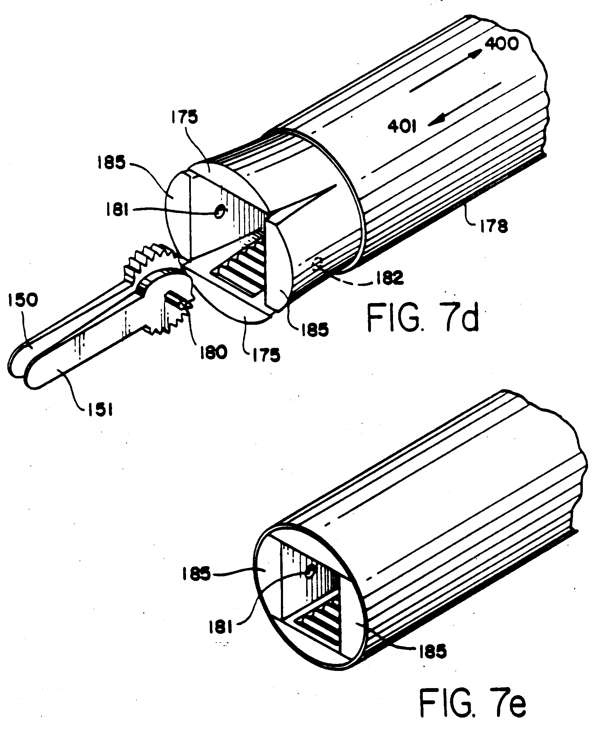

Figure 7 (a), (b), and (c) illustrate several different means of actuating elongate elements. In Figure 7 (a), elongate elements 150 and 151 are joined together at pivot 152. Also joined at pivot 152 is one end of a linkage composed of four bars 153, 154, 155, and 156, which are pivotally connected to each other. Elongate elements 150 and 151 are preferably rigidly attached to bars 153 and 154, respectively. Alternatively, bars 153 and 154 may merely represent extensions of elongate elements 150 and 151, respectively. Pivot 152 is preferably fixed to a cannula 159. The pivot 157 at the other end of the four-bar linkage is joined to rod 158. When rod 158 is pushed in direction 301, pivot 157 is pushed closer to pivot 152. This will cause elongate elements 150 and 151 to splay apart. Since the transverse dimension of four-bar linkage 153, 154, 155, and 156 which is perpendicular to rod 158 becomes larger as pivot 157 approaches pivot 152, slots 160 and 161 may be provided in cannula 159 to permit pivot 157 to approach closer to pivot 152 if the transverse dimension of cannula 159 is small. Rod 158 may be pushed (or pulled) relative to cannula 159 by any suitable manually operated mechanism. Examples of manually operated mechanisms include sliders, pistol grip handles, scissors handles, and syringe-plunger arrangements.

Elongate elements 150 and 151 may be constrained in deformed and straightened shapes within a sheath 162. This will permit compact and relatively atraumatic entry into a body. Rod 158 can then be used to pushed axially in direction 301 within sheath 162. The four-bar linkage 153, 154, 155, and 156 will partially extend through slots 160 and 161 in cannula 159, but the inner surface of sheath 162 will prevent pivot 157 from fully approaching pivot 152. Therefore, cannula 159 will be forced to move in direction 301, and elongate elements 150 and 151 will extend from the end of sheath 162 in direction 301. In their extended position, elongate elements 150 and 151 will not be constrained, and they may recover toward their preset curved shape. However, slots 163 and 164 are provided in sheath 162 to permit rod 158 to push pivot 157 fully toward pivot 152 in order to fully splay elongate elements 150 and 151 apart. Slots 163 and 164 in sheath 162 may be made to overlap slots 160 and 161 in cannula 159 by simply extending cannula 159 far enough within sheath 162, or by extending cannula 159 far enough within sheath 162 and then rotating sheath 162 relative to cannula 159 to allow the respective slots to coincide. Rod 158 may then be used to splay or increasingly overlap elongate elements 150 and 151 as desired.

Rod 158 can be moved in direction 302 so that pivot 157 is moved as far away as possible from pivot 152. This will cause elongate elements 150 and 151 to be in their most overlapped configuration. Moving rod 158 further in direction 302 will cause cannula 159 to slide in direction 302, and will cause elements 150 and 151 to be drawn into deformed and straightened shapes within sheath 162. This will permit the entire assembly to be withdrawn from the body in a compact and relatively atraumatic fashion. The passive member of the manually operated mechanism would preferably be mounted to sheath 162.

In this fashion, the extension and withdrawal of elongate elements 150 and 151 from or into sheath 162 can be accomplished by utilizing an expanded stroke of the same manually operated mechanism which is used to splay or increasingly overlap elongate elements 150 and 151. In this case, a means must be provided to prevent cannula 159 from sliding beyond a certain location within sheath 162 in direction 301. Also, a means may be provided to minimize relative motion between cannula 159 and sheath 162 while the four-bar linkage is being used to repeatedly move elongate elements 150 and 151 toward their splayed or overlapped configurations. Furthermore, the manually operated mechanism would preferably permit axial rotation of the entire assembly of sheath 162 and its contents relative to the manually operated mechanism, so that elongate elements 150 and 151 can be oriented in any desired direction relative to the manually operated mechanism.

In the configuration illustrated in Figure 7 (a), it will be noted that movement of rod 158 in direction 301 will tend to splay elongate elements 150 and 151 apart. As described above, one method of minimizing this splaying before the device is in the correct location is to create slots in specific locations of sheath 162. In an alternative method, bars 156 and 155 are shorter than bars 153 and 154, and pivot 157 is already positioned as close as possible to pivot 152 during placement of the device into a body. (In this configuration, bars 155 and 156 would overlap bars 153 and 154, respectively.) Moving rod 158 in direction 301 will then urge elongate elements 150 and 151 toward their overlapped configuration, even though the elongate elements can be extended beyond the end of sheath 162 by the same motion in direction 301 when the sheath is held fixed. Elongate elements 150 and 151 can then be splayed apart by moving rod 158 in direction 302. When the device is to be withdrawn from a body, rod 158 is moved further in direction 302, so that pivot 157 is as far as possible from pivot 152, where the configuration shown in Figure 7 (a) would be an intermediate position. Elongate elements 150 and 151 will thereby be urged back toward their overlapped configuration. Moving rod 158 even further in direction 302, relative to sheath 162, will permit withdraw elongate elements 150 and 151 into sheath 162.

Figure 7 (b) shows an embodiment in which elongate elements 150 and 151 have a pivot 165 and body portions 166 and 167, respectively. Body portions 166 and 167 have slots 168 and 169, respectively. A rod 190 has a peg 191 which is oriented to slideably engage slots 168 and 169. Pivot 165 is fixed to the cannula 192, and slots 168 and 169 are preferably oriented so that motion of rod 190 in direction 310 will urge elongate elements 150 and 151 toward their overlapped configuration, and motion of rod 190 in direction 320 will splay elongate elements 150 and 151 apart. Cannula 192 may be substantially the same as cannula 159 shown in Figure 7 (a). In addition, a sheath 193, which may substantially be the same as sheath 162 shown in Figure 7 (a), can be utilized. The function and use of the embodiment shown in Figure 7 (b) is then substantially the same as the embodiment shown in Figure 7 (a).

A variation of the embodiment illustrated in Figure 7 (b) would include elongate elements in which the slots are placed distal to the pivot point between the elongate elements. (That is, the slots are located between the pivot point and the tips of the elongate elements). Body portions 166 and 167 as shown in Figure 7 (b), and slots 160, 161, 163, and 164 as shown in Figure 7 (a) may then not be necessary. However, the actuating rod (such as rod 190 shown in Figure 7 (b)), would have to be designed so that it does not interfere with the pivot point between the elongate elements.

Figure 7 (c) shows another embodiment in which the elongate elements 150 and 151 may be made to splay apart or increasingly overtap each other. Elongate elements 150 and 151 are hinged at pivot 170, which is preferably fixed to a cannula 176. Surrounding pivot 170, elongate elements 150 and 151 each have a rounded body portion with teeth along edges 171 and 172, respectively. The teeth engage the corresponding grooves located in jaws 173 and 174 of sliding member 175. The degree of splaying or overlapping of elongate elements 150 and 151 may be limited by limiting the lengths of edges 171 or 172 which are toothed. Additionally, or alternatively, the degree of splaying or overlapping of elongate elements 150 and 151 may be limited by limiting the lengths of the grooved zones in jaws 173 and 174. Sliding member 175 is moved in direction 303 or 305 by any suitable manually operated mechanism. Examples of manually operated mechanisms include sliders, pistol grip handles, scissors handles, and syringe-plunger arrangements. Elongate elements 150 and 151 are preferably moved toward their overlapped configuration when sliding member 175 is moved in direction 303 and moved toward their splayed apart configuration when sliding member 175 is moved in direction 305. However, toothed edges 171 and 172 can be located on elongate elements 150 and 151 such that moving sliding member 175 in direction 303 moves elongate elements 150 and 151 toward their splayed configuration and moving sliding member 175 in direction 305 moves elongate elements 150 and 151 toward their overlapped configuration.

Elongate elements 150 and 151 may be constrained in deformed and straightened shapes within a sheath 178. This will permit compact and relatively atraumatic entry into a body. Sliding member 175 can then be moved in direction 303 relative to sheath 178 in order to extend elongate elements 150 and 151 from the end of the sheath. In the preferred mode, this motion will also tend to keep elongate elements 150 and 151 in their overlapped configuration without splaying these elements apart in the wrong direction. (As described above, toothed edges 171 and/or 172 and/or the jaws 173 and/or 174 can be designed to prevent splaying in the wrong direction). Elongate elements 150 and 151 can then be repeatedly moved toward their splayed configuration or their overlapped configuration by moving sliding member 175 in directions 305 or 303, respectively, and a means may be provided to minimize relative motion between cannula 176 and sheath 178 during this repetitive motion.

Elongate elements 150 and 151 can be withdrawn back inside sheath 178 by forcibly moving sliding member 175 in direction 305 relative to sheath 178. The end of sheath 178 will force elongate elements 150 and 151 into their overlapped configuration, as well as forcing elongate elements 150 and 151 into straightened and deformed shapes into sheath 178 in order to permit the entire assembly to be withdrawn from a body in a compact and relatively atraumatic fashion. Alternatively, sheath 178 can be extended over elongate elements 150 and 151 to straighten and deform these elements into sheath 178 and to permit the entire assembly to be withdrawn from a body in a compact and relatively atraumatic fashion.

If a sheath 178 is utilized, it would preferably be mounted to the passive member of the manually operated mechanism. In this fashion, the extension and withdrawal of elongate elements 150 and 151 from or into sheath 178 can be accomplished by utilizing an expanded stroke of the same manually operated mechanism which is used to move sliding member 175 in order to splay or increasingly overlap elongate elements 150 and 151.

When elongate elements 150 and 151 are to be removed and replaced, it would be advantageous to move cannula 176 far enough in direction 303 so that pivot 170 is beyond the end of sheath 178. Then the pivot pin can be removed, sliding member 175 can be extended beyond the end of cannula 176, and elongate elements 150 and 151 can be simply slid out of jaws 173 and 174 in a direction perpendicular to the axis of sliding member 175. In addition, in order to permit the elongate elements 150 and 151 to be oriented in any desired direction relative to the manually operated mechanism, this mechanism would preferably permit axial rotation of the entire assembly of sheath 178 and its contents relative to the actuating mechanism.

Figure 7 (d) shows how sliding member 175 could be configured around a pivot fixing member 185, which has holes 180 and 182. Elongate elements 150 and 151 are rotatably mounted on a pin 180. The ends of pin 180 can be placed into holes 181 and 182 when sheath 178 is pulled back in direction 400, since the ends of sliding member 175 and the ends of pivot fixing member 185 can gently splay apart when they are not held within sheath 178. When sheath 178 is moved back in direction 401, elongate elements 150 and 151 will be securely held when pin 180 is within sheath 178. The end of pivot fixing member 185 which has holes 181 and 182 can be fork shaped. Preferably a means is provided which minimizes motion of pivot fixing member 185 relative to sheath 178 when sliding member 175 is utilized to repeatedly move elongate elements 150 and 151 toward their splayed or overlapped configurations. Figure 7 (e) shows the device before sheath 178 is pulled back to permit insertion of elongate elements 150 and 151.

In the embodiments described for Figures 7 (a), (b), (c), and (d), the elongate elements are preferably made of a pseudoelastic shape memory alloy. Also, in any of the embodiments described for Figures 7 (a), (b), (c), and (d), the elongate elements can be used for cutting, grasping, and/or separating tissues. The end portions of the elongate elements can be fashioned appropriately for any of these functions, or separate appropriately designed parts may be attached to the end portions of the elongate elements.

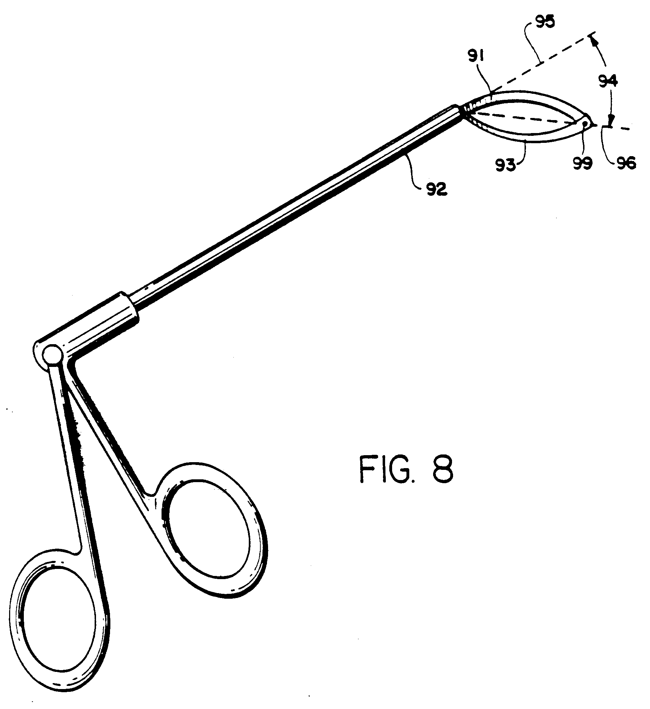

Figure 8 shows a cutting device, similar to the device shown in Figure 1, with curved elongate elements 91 and 93 extended from a housing 92. This permits the elongate elements to be both open for cutting or grasping and curved at an angle 94 away from axis 95 of housing 92. Angle 94 is defined by the axis 95 of housing 92 and the straight line 96 created between the point of intersection of axis 95 with the distal end of housing 92 and the pin 99. Angle 94 can be any desired angle, even greater than 90 degrees, thus permitting cutting or grasping in a direction off axis 95. This provides access to difficult to reach locations in the body. Elongate elements 91 and 93 are shaped so that they circumscribe spherical arcs which allow the elements to engage each other and perform the cutting or grasping function, either as they are retracted back into housing 92, or as housing 92 is extended over the elongate elements. The portions of elongate elements 91 and 93 which enter housing 92 assume a less curved shape. Since elongate elements 91 and 93 may be made of pseudoelastic shape memory alloy, they may attain these less curved shapes by undergoing some phase transition from austenite to stress-induced-martensite.

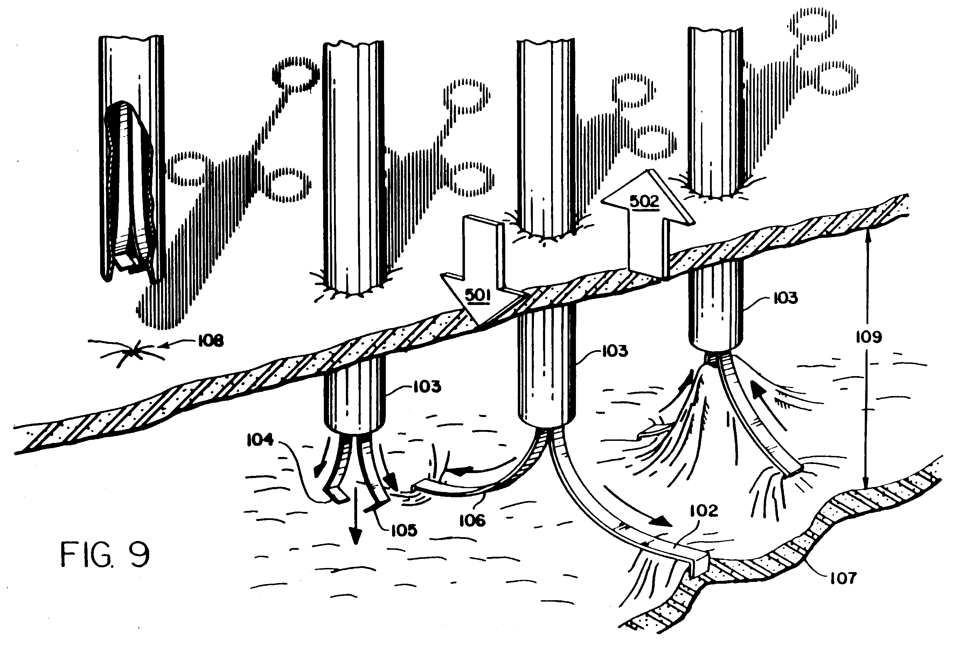

Figure 9 shows a device in which elongate elements 102 and 106, preferably made of pseudoelastic shape memory alloy, are first held constrained in straightened and deformed shapes inside a cannula 103. This permits compact placement into a body through tissue incision or orifice 108. Elongate elements 102 and 106 are then extended out of cannula 103 by moving elongate elements 102 and 106 in direction 501 relative to cannula 103. Since at least part of extended elongate elements 102 and 106 are no longer constrained, they will splay apart due to recovery of the pseudoelastic shape memory alloy into its preset curved unconstrained shapes. Cannula 103 can be then be extended onto elongate elements 102 and 106 to force these elements to approach each other. Alternatively, elongate elements 102 and 106 can be withdrawn back into cannula 103 to force these elements to approach each other. In either mode, the tips of elongate elements 102 and 106 can be used to grasp tissue 107 or an object. The grasping function of elongate elements 102 and 106 can be enhanced by providing the end portions of these elements with bends 104 and 105, teeth, or the like at their tips. Elongate elements 102 and 106 may also be ribbed or toothed along their entire lengths. The described mode of action may permit the instrument to be used multiple times in each location.
The large pseudoelastic deformation of shape memory alloys (up to 10%) permits much wider splaying of elongate elements 102 and 106 over a much shorter distance 109 than would be possible with traditional metals. This permits working in confined spaces, particularly in endoscopic or laparoscopic surgery. A variation of this embodiment may include more than two elongate elements.

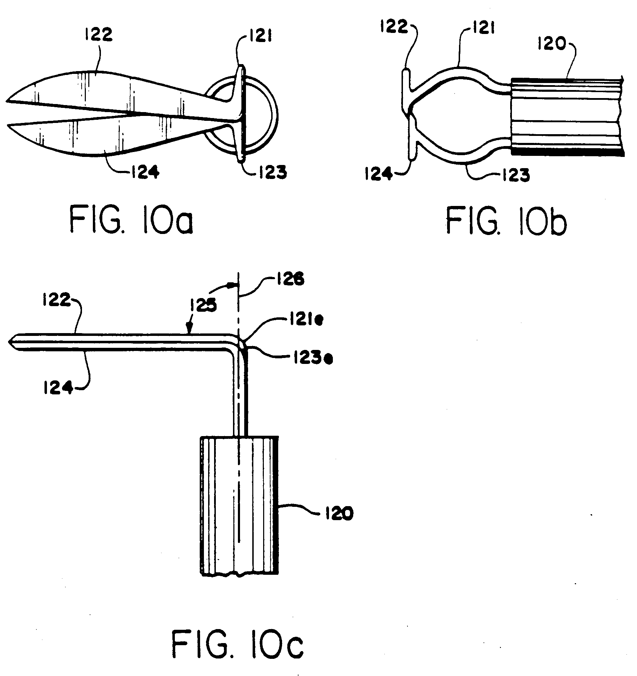

Figures 10 (a), (b), and (c) illustrate three views of another embodiment. As elongate elements 121 and 123 are extended outside the housing 120, they splay outward causing end portions 122 and 124 to separate also. When elongate elements 121 and 123 are partially withdrawn into housing 120, they cause end portions 122 and 124 to approach each other. If elongate elements 121 and 123 are further withdrawn into housing 120, the sections 121e and 123e of elongate elements 121 and 123 are forced to deform into straightened shapes in order to pass into housing 120, thus causing the direction of orientation of end portions 122 and 124 to approach the direction of axis 126 of housing 120, and the angle 125 approaches zero degrees (angle 125 is defined by axis 126 of housing 120 and the plane of end portions 122 and 124). End portions 122 and 124 may also be fully or partially withdrawn into housing 120, if desired. The straight configuration permits easy removal of the instrument from a body in a compact and relatively atraumatic fashion. However, with elongate elements 121 and 123 in a completely extended position, angle 125 permits access to difficult to reach locations.

In the embodiments shown in Figures 10 (a), (b), and (c), the body portions of elongate elements 121 and 123 are preferably made of pseudoelastic shape memory alloy. Alternatively, sections 121e and 123e may be the only parts of elongate elements 121 and 123 which are made of pseudoelastic shape memory alloy. End portions 122 and 124 may also be made of pseudoelastic shape memory alloy, but they could be made of any suitable material, even if elements 121 and 123 are made at least in part of pseudoelastic shape memory alloy. End portions 122 and 124 may have a cutting function or a grasping function. They may also be used to separate tissues. The described mode of action may permit the instrument to be used multiple times in each location.

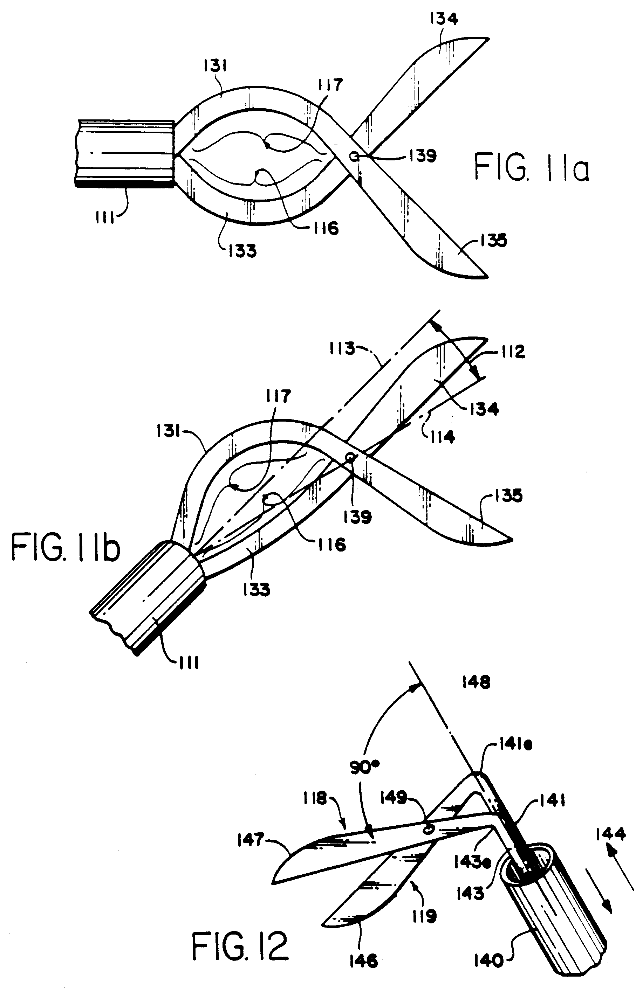

Figures 11 (a) and 11 (b) show embodiments similar to the embodiments shown in Figures 1 and 8, respectively. In Figures 12 (a) and 12(b), the elongate elements 131 and 133 extend beyond the pin 139 in order to provide end portions 134 and 135. End portions 134 and 135 may be unitary extensions of elongate elements 131 and 133 or they may be separate portions bolted or attached to elongate elements 131 and 133. The action of withdrawing elongate elements 131 and 133 into housing 111 closes and deforms body portions 116 and 117, and the scissor action is transmitted to end portions 134 and 135. In this manner, the body portions of the elongate elements act as the actuating means for the end portions of the elongate elements. Figure 12 (b) illustrates a curved version of Figure 11 (a). The angle 112 is defined by the axis 113 of housing 111 and the straight line 114 created between the point of intersection of axis 113 with the distal end of the housing and pin 139. Angle 112 can be any number of degrees, even greater than 90 degrees, thus permitting cutting or grasping in a direction off axis 113. This provides access to difficult to reach locations within a body.

In the embodiments of Figures 11 (a) and 11 (b), body portions 116 and 117 are preferably made of pseudoelastic shape memory alloy. Alternatively, only end portions 134 and 135 may be made of pseudoelastic shape memory alloy, but these end portions could be made of any suitable material, even if body portions 116 and 117 are made of pseudoelastic shape memory alloy. End portions 134 and 135 may have a cutting function or a grasping function. They may also be used to separate tissues. The described mode of action may permit the instrument to be used multiple times in each location.

Figure 12 illustrates another embodiment similar to the embodiment shown in Figure 11 (b). Body portions 141 and 143 of elongate elements 118 and 119 are used to create both a scissors action through a pinned location 149 and also to provide the ability to direct the scissor action at an angle of about ninety degrees off the axis 148 of housing 140. Elements 141 and 143 splay apart when they are outside of housing 140. As housing 140 is pushed over the body portions 141 and 143 in direction 144, sections 141e and 143e move toward one another. This action in turn causes the end portions 146 and 147 to approach each other in a scissor fashion by pivoting around pin 149. Because the relative movement of housing 140 in directions 144 and 145 is perpendicular to end portions 146 and 147, the position of these end portions is unchanged with respect to the tissue location. After end portions 146 and 147 have closed, further withdrawal of elongate elements 118 and 119 into housing 140 causes sections 141e and 143e to straighten from their curved shapes. This permits end portions 146 and 147 to generally align with axis 148 of housing 140. End portions 146 and 147 may also be fully or partially withdrawn into housing 140, if desired. The straight configuration permits easy removal of the instrument from a body in a compact and relatively atraumatic fashion.

In the embodiments of Figure 12, body portions 141 and 143 of elongate elements 118 and 119 are preferably made of pseudoelastic shape memory alloy. Alternatively, sections 141e and 143e may be the only parts of body portions 141 and 143 which are made of pseudoelastic shape memory alloy. End portions 146 and 147 may also be made of pseudoelastic shape memory alloy, but they could be made of any suitable material, even if body portions 141 and 143 are made at least in part of pseudoelastic shape memory alloy. End portions 146 and 147 may have a cutting function or a grasping function. They may also be used to separate tissues. The described mode of action may permit the instrument to be used multiple times in each location.

A variation of the embodiment shown in Figure 12 would have end portions 146 and 147 in a plane which is parallel to axis 148, so that pivot 149 is on an axis which is perpendicular to axis 148. In this embodiment, moving body portion 141 in direction 144 and/or moving body portion 143 in direction 145 would tend to splay end portions 146 and 147 apart. Moving body portion 141 in direction 145 and/or moving element 143 in direction 144 would tend to bring end portions 146 and 147 into a more overlapped configuration. In this manner, the body portions of the elongate elements act as the actuating means for the end portions of the elongate elements. In order to facilitate the requisite bending in sections 141e and 143e, body portions 141 and 143 would preferably be either round or made of flat material oriented in a plane perpendicular to the plane of end portions 146 and 147. If body portions 141 and 143 are made of flat material, they may include a 90 degree twist in the material between sections 141e and 143e and end portions 146 and 147, respectively.

## Claims

1. A device for grasping or cutting an object which comprises
(a) at least two elongate elements (1,3; 21,23; 51,52; 150,151; 91,93; 102,106; 121,123; 118,119), positioned alongside one another, each having a body portion (7), and an end portion (5,29; 122,124; 134,135;), the end portions of the elements:
(i) being capable of being splayed outwardly apart from one another when free of transverse constraint and presenting grasping or cutting surfaces to an object to be grasped or cut that is placed between them; and
(ii) being capable of being moved inwardly towards one another to grasp or cut said objected; and
(b) actuating means (158; 190; 175, Figs 8+9 - not numbered,)
characterised in that a portion of at least one of the elements and/or of said actuating means is formed from a material which exhibits pseudoelasticity when deformed under an applied stress.

2. A device according to claim 1 which further comprises
(c) a hollow elongate component (11; 25; 53,55; 159; 192; 176; 178; 92; 103; 120; 111; 140), preferably a polymeric or metallic tube, in which at least parts of the said elements are positioned in use so that the elements and the component are longitudinally slidable relative to one another so that at least the end portions of the elements can be slid into and out of said component and wherein a portion of at least one of the elements is formed from a material which exhibits pseudoelasticity when deformed under an applied stress and is pseudoelastically deformed when positioned within the component.

3. A device as claimed in claim 1 or 2 wherein the pseudoelastic material is a shape memory alloy.

4. A device as claimed in any preceding claim, in which the end portion of at least one, preferably both, of the elements is formed from a shape memory alloy which exhibits pseudoelasticity.

5. A device as claimed in clam 4, wherein said end portions are (a) splayed apart (Figs 4b, 4c, 7a, 8, 11, 12, Fig 1, 3b, 3c) when not deformed within the hollow component and are moved toward one another (Fig. 3e, 4a, 7c) when drawn into the hollow component or when the hollow component is drawn over said end portions;
and/or are (b) curved at a predetermined angle (94) with respect to the hollow component (Fig 8, 92, 10c) when said end portion(s) is (are) extruded from the component, or (c) straight (95) and at a predetermined angle with respect to the hollow component when said end portion(s) is (are) extruded from the component.

6. A device as claimed in any preceding claim, wherein the actuating means (158, 190, 175) is arranged for splaying the end portions of the elements apart from one another, and/or moving said end portions toward one another, and/or sliding the elements with respect to the hollow component (Fig 7,) and/or rotating the elements with respect to the hollow component (Fig 8).

7. A device as claimed in any preceding claim in which said actuating means is formed from a shape memory alloy.

8. A device as claimed in any preceding claim in which at least one of the end portion, preferably both, and/or at least one of the body portions, preferably both, of the elongate elements of the device are made of a material other than a shape memory alloy.

9. A device as claimed in any of claims 1 to 6, in which said actuating means is formed from a material other than a shape memory alloy.

10. A device as claimed in any of claim 1 to 6 and 9 in which at least one of the end portions, preferably both, and/or at least one of the body portions, preferably both, of the elongate elements of the device are made of a shape memory alloy.

11. A device as claimed in any proceeding claim, which further comprises a cutting edge of a material other than a shape memory alloy, preferably stainless steel, preferably said cutting edge extending beyond the end portion of at least one of the elements.

12. A device as claimed in any preceding claim, in which the end portions of the elements are pivotally connected to one other towards their free ends (Fig 1(9); Fig 4(31); fig 8(99)).

13. A device as claimed in any preceding claim, which includes a flexible component that extends between the end portions of the elements so as, together with the end portions of the elements, to form a closed loop (Figs 1 + 8).

14. A device as claimed in any preceding claim, in which the body portions of the elements are attached to one another (Figs 4, 11, 12).

15. A device as claimed in any preceding claim, in which at least the end portions of the elements are readily removable from the device.

16. A device according to any preceding claim, wherein the pseudoelastic material exhibits pseudoelasticity at or around body temperature.

17. A device according to any preceding claim adapted for surgical insertion into a human or animal body in which access to the object to be grasped or cut within the body is restricted.

18. An assembly which comprises:
(a) at least two elongate elements (1,3; 21,23;, 51,52; 150,151; 91,93; 102,106; 121,123; 118,119), positioned alongside one another, each having a body portion (7) and an end portion (5; 29; 122,124; 134,135;), the end portions of the elements:
(i) being capable of being splayed outwardly apart from one another when free of transverse constraint and presenting grasping or cutting surfaces to an objected to be grasped or cut that is placed between them in use; and
(ii) being capable of being moved inwardly towards one another to grasp or cut said object;
characterised in that a portion of at least the assembly is suitable for use in a device according to any preceding claim, and in that a portion of at least one of the elements is formed from a material which exhibits pseudoelasticity when deformed under an applied stress.

## Patentansprüche

1. Vorrichtung zum Ergreifen oder Schneiden eines Objekts, die folgendes aufweist:
(a) wenigstens zwei langgestreckte Elemente (1, 3; 21, 23; 51, 52; 150, 151; 91, 93; 102, 106; 121, 123; 118, 119), welche in Längsrichtung nebeneinander angeordnet sind und welche jeweils einen Körperbereich (7) und einen Endbereich (5, 29; 122, 124; 134, 135) aufweisen, wobei die Endbereiche der Elemente
(i) in der Lage sind, sich voneinander weg nach außen zu spreizen, wenn kein transversaler Zwang auf sie einwirkt, und einem zu ergreifenden oder zu schneidenden Objekt, welches zwischen ihnen plaziert ist, Greif- oder Schneidflächen zu bieten; und
(ii) welche in der Lage sind, sich nach innen aufeinander zu zu bewegen, um das Objekt zu ergreifen oder zu schneiden; und
(b) eine Betätigungseinrichtung (158; 190; 175, Fig. 8 und 9 - nicht beziffert),
dadurch gekennzeichnet,
daß ein Bereich von wenigstens einem der Elemente und/oder der Betätigungseinrichtung aus einem Material gebildet ist, welches eine Pseudoelastizität zeigt, wenn es unter einer aufgebrachten Belastung verformt wird.

2. Vorrichtung nach Anspruch 1,
welche ferner folgendes aufweist:
(c) eine hohle langgestreckte Komponente (11; 25; 53, 55; 159; 192; 176; 178; 92; 103; 120; 111; 140), vorzugsweise ein polymeres oder metallisches Rohr, in welchem zumindest Teile der Elemente im Gebrauch angeordnet sind, so daß die Elemente und die Komponente in Längsrichtung relativ zueinander verschiebbar sind, so daßzumindest die Endbereiche der Elemente in die Komponente hinein und aus der Komponente heraus gleiten können, und wobei ein Bereich von wenigstens einem der Elemente aus einem Material gebildet ist, welches eine Pseudoelastizität zeigt, wenn es unter einer aufgebrachten Belastung verformt wird, und welches pseudoelastisch verformt wird, wenn es innerhalb der Komponente positioniert wird.

3. Vorrichtung nach Anspruch 1 oder 2,
wobei das pseudoelastische Material eine Legierung mit Formerinnerungsvermögen ist.

4. Vorrichtung nach einem der vorhergehenden Ansprüche, bei der die Endbereiche von wenigstens einem, vorzugsweise von beiden Elementen aus einer Legierung mit Formerinnerungsvermögen gebildet sind, welche eine Pseudoelastizität besitzt.

5. Vorrichtung nach Anspruch 4,
wobei die Endbereiche
(a) auseinandergespreizt sind (Fig. 4b, 4c, 7a, 8, 11, 12, Fig. 1, 3b, 3c), wenn sie nicht innerhalb der hohlen Komponente verformt sind und aufeinander zu bewegt werden (Fig. 3e, 4a, 7c), wenn sie in die hohle Komponente hineingezogen werden oder wenn die hohle Komponente über die Endbereiche hinüber gezogen wird; und/oder (b) unter einem vorbestimmten Winkel (94) in Bezug auf die hohle Komponente (Fig. 8, 92, 10c) gekrümmt sind, wenn der/die Endbereich(e) aus der Komponente herausgefahren ist/sind;
oder (c) gerade und unter einem vorbestimmten Winkel in Bezug auf die hohle Komponente verlaufen, wenn der/die Endbereich(e) aus der Komponente herausgefahren ist/sind.

6. Vorrichtung nach einem der vorhergehenden Ansprüche,
wobei die Betätigungseinrichtung (158, 190, 175) so angeordnet ist, daß sie die Endbereiche der Elemente voneinander wegspreizt, und/oder die Endbereiche aufeinander zu bewegt, und/oder die Elemente relativ zur hohlen Komponente (Fig. 7) gleiten läßt, und/oder die Elemente relativ zu der hohlen Komponente (Fig. 8) dreht.

7. Vorrichtung nach einem der vorhergehenden Ansprüche,
bei der die Betätigungseinrichtung aus einer Legierung mit Formerinnerungsvermögen gebildet ist.

8. Vorrichtung nach einem der vorhergehenden Ansprüche,
bei welcher wenigstens der eine der Endbereiche, vorzugsweise beide und/oder wenigstens der eine der Körperbereiche, vorzugsweise beide, der langgestreckten Elemente der Vorrichtung aus einem anderen Material als einer Legierung mit Formerinnerungsvermögen bestehen.

9. Vorrichtung nach einem der Ansprüche 1 bis 6,
bei der die Betätigungseinrichtung aus einem anderen Material als einer Legierung mit Formerinnerungsvermögen besteht.

10. Vorrichtung nach einem der Ansprüche 1 bis 6 und 9,
bei der wenigstens der eine der Endbereiche, vorzugsweise beide Endbereiche und/oder wenigstens der eine der Körperbereiche, vorzugsweise beide Körperbereiche der langgestreckten Elemente der Vorrichtung aus einer Legierung mit Formerinnerungsvermögen bestehen.

11. Vorrichtung nach einem der vorhergehenden Ansprüche,
welche ferner eine Schneidkante aus einem anderen Material als einer Legierung mit Formerinnerungsvermögen, vorzugsweise aus rostfreiem Stahl aufweist, wobei die Schneidkante sich vorzugsweise über den Endbereich von wenigstens dem einen der Elemente hinaus erstreckt.

12. Vorrichtung nach einem der vorhergehenden Ansprüche,
bei welcher die Endbereiche der Elemente zu ihren freien Enden hin drehbar miteinander verbunden sind (Fig. 1(9); Fig. 4(31); Fig. 8(99)).

13. Vorrichtung nach einem der vorhergehenden Ansprüche,
welche eine flexible Komponente aufweist, welche sich zwischen den Endbereichen der Elemente derart erstreckt, daß sie zusammen mit den Endbereichen der Elemente eine geschlossene Schleife (Fig. 1 + 8) bildet.

14. Vorrichtung nach einem der vorhergehenden Ansprüche,
bei der die Körperbereiche der Elemente aneinander angebracht sind (Fig. 4, 11, 12).

15. Vorrichtung nach einem der vorhergehenden Ansprüche,
bei der wenigstens die Endbereiche der Elemente leicht von der Vorrichtung entfernbar sind.

16. Vorrichtung nach einem der vorhergehenden Ansprüche,
bei der das pseudoelastische Material bei der oder in der Größenordnung von der Körpertemperatur eine Pseudoelastizität besitzt.

17. Vorrichtung nach einem der vorhergehenden Ansprüche,
die zum Einführen zu chirurgischen Zwecken in einen menschlichen oder tierischen Körper geeignet ist, in dem der Zugang zu dem innerhalb des Körpers zu ergreifenden oder zu schneidenden Objekt eingeschränkt ist.

18. Anordnung, die folgendes aufweist:
(a) wenigstens zwei langgestreckte Elemente (1, 3; 21, 23; 51, 52; 150, 151; 91, 93; 102, 106; 121, 123; 118, 119), welche in Längsrichtung nebeneinander angeordnet sind und welche jeweils einen Körperbereich (7) und einen Endbereich (5; 29; 122, 124; 134, 135) aufweisen, wobei die Endbereiche der Elemente
(i) in der Lage sind, sich voneinander weg nach außen zu spreizen, wenn kein transversaler Zwang auf sie einwirkt, und einem zu ergreifenden oder zu schneidenden Objekt, welches sich im Gebrauch zwischen ihnen befindet, Greif- oder Schneidflächen zu bieten; und
(ii) welche in der Lage sind, sich nach innen aufeinander zu zu bewegen, um das Objekt zu ergreifen oder zu schneiden;
dadurch gekennzeichnet,
daß ein Bereich von zumindest der Anordnung zum Gebrauch in einer Vorrichtung nach einem der vorhergehenden Ansprüche geeignet ist
und daß ein Bereich von mindestens dem einen der Elemente aus einem Material gebildet ist, welches eine Pseudoelastizität besitzt, wenn es unter einer aufgebrachten Belastung verformt wird.

## Revendications

1. Dispositif pour saisir ou couper un objet, qui comprend
(a) au moins deux éléments allongés (1, 3 ; 21, 23 ; 51, 52 ; 150, 151 ; 91, 93 ; 102, 106 ; 121, 123 ; 118, 119) positionnés le long l'un de l'autre, chacun ayant une partie de corps (7) et une partie d'extrémité (5, 29 ; 122, 124 ; 134, 135), les parties d'extrémité des éléments :
(i) étant capables d'être déployées vers l'extérieur, l'une à l'écart de l'autre, lorsqu'elles ne sont pas soumises à une contrainte transversale, et présentant des surfaces de préhension ou coupantes à un objet devant être saisi ou coupé qui est placé entre elles ; et
(ii) étant capables d'être déplacées vers l'intérieur, l'une vers l'autre, pour saisir ou couper ledit objet ; et
(b) des moyens d'actionnement (158 ; 190 ; 175, figures 8+9 - non numérotées),
caractérisé en ce qu'une partie d'au moins un des éléments et/ou desdits moyens d'actionnement est formé en un matériau qui présente une pseudo-élasticité lorsqu'il est déformé par une contrainte appliquée.

2. Dispositif selon la revendication 1, qui comprend en outre :
(c) un composant allongé creux (11 ; 25 ; 53, 55 ; 192 ; 176 ; 178 ; 92 ; 103 ; 120 ; 111 ; 140), préférablement un tube polymère ou métallique, dans lequel au moins des parties desdits éléments sont positionnées en utilisation de sorte que les éléments et le composant peuvent coulisser longitudinalement les uns par rapport aux autres, de sorte qu'au moins les parties d'extrémité des éléments peuvent être mises en coulissement dans ledit, et en dehors dudit, composant, et dans lequel une partie d'au moins un des éléments est formée en un matériau qui présente une pseudo-élasticité lorsqu'il est déformé par une contrainte appliquée et est déformée pseudo-élastiquement lorsqu'elle est positionnée dans le composant.

3. Dispositif selon la revendication 1 ou 2, dans lequel le matériau pseudo-élastique est un alliage à mémoire de forme.

4. Dispositif selon l'une quelconque des revendications précédentes, dans lequel la partie d'extrémité d'au moins un élément, préférablement les deux éléments, est formée en un alliage à mémoire de forme qui présente une pseudo-élasticité.

5. Dispositif selon la revendication 4, dans lequel lesdites parties d'extrémité sont (a) déployées (figures 4b, 4c, 7a, 8, 11, 12, figures 1, 3b, 3c) lorsqu'elles ne sont pas déformées dans le composant creux et sont déplacées l'une vers l'autre (figure 3e, 4a, 7c) lorsqu'elles sont tirées dans le composant creux ou lorsque le composant creux est tiré par-dessus lesdites parties d'extrémité ;
et/ou sont (b) incurvées selon un angle prédéterminé (94) par rapport au composant creux (figure 8, 92, 10c) lorsque la (les) partie(s) d'extrémité est (sont) extrudée(s) à partir du composant, ou (c) est (sont) droite(s) (95) et selon un angle prédéterminé par rapport au composant creux lorsque la (les) partie(s) d'extrémité est (sont) extrudée(s) à partir du composant.

6. Dispositif selon l'une quelconque des revendications précédentes, dans lequel des moyens d'actionnement (158, 190, 175) sont disposés pour déployer les parties d'extrémité des éléments l'une à l'écart de l'autre, et/ou déplacer lesdites parties d'extrémité l'une vers l'autre, et/ou faire coulisser les éléments par rapport au composant creux (figure 7) et/ou faire tourner les éléments par rapport au composant creux (figure 8).

7. Dispositif selon l'une quelconque des revendications précédentes, dans lequel lesdits moyens d'actionnement sont formés en un alliage à mémoire de forme.

8. Dispositif selon l'une quelconque des revendications précédentes, dans lequel au moins une des parties d'extrémité, préférablement les deux, et/ou au moins une des parties de corps, préférablement les deux, des éléments allongés du dispositif sont fabriquées en un matériau autre qu'un alliage à mémoire de forme.

9. Dispositif selon l'une quelconque des revendications 1 à 6, dans lequel lesdits moyens d'actionnement sont formés en un matériau autre qu'un alliage à mémoire de forme.

10. Dispositif selon l'une quelconque des revendications 1 à 6 et 9, dans lequel au moins une des parties d'extrémité, préférablement les deux, et/ou au moins une des parties de corps, préférablement les deux, des éléments allongés du dispositif sont fabriquées en alliage à mémoire de forme.

11. Dispositif selon l'une quelconque des revendications précédentes, qui comprend en outre un bord coupant en un matériau autre qu'un alliage à mémoire de forme, préférablement en acier inoxydable, ledit bord coupant préférablement s'étendant au-delà de la partie d'extrémité d'au moins un des éléments.

12. Dispositif selon l'une quelconque des revendications précédentes, dans lequel les parties d'extrémité des éléments sont connectées en pivotement l'une à l'autre vers leurs extrémités libres (figure 1 (9) ; figure 4 (31) ; figure 8 (99)).

13. Dispositif selon l'une quelconque des revendications précédentes, qui comprend un composant flexible qui s'étend entre les parties d'extrémité de l'élément de façon à, avec les parties d'extrémité des éléments, former une boucle fermée (figures 1 + 8).

14. Dispositif selon l'une quelconque des revendications précédentes, dans lequel les parties de corps des éléments sont fixées l'une à l'autre (figures 4, 11, 12).

15. Dispositif selon l'une quelconque des revendications précédentes, dans lequel au moins les parties d'extrémité des éléments peuvent être facilement retirées du dispositif.

16. Dispositif selon l'une quelconque des revendications précédentes, dans lequel le matériau pseudoélastique présente une pseudo-élasticité à, ou autour de, la température du corps.

17. Dispositif selon l'une quelconque des revendications précédentes, adapté pour une insertion chirurgicale dans un corps humain ou un corps d'animal, dans lequel l'accès à l'objet devant être saisi ou coupé dans le corps, est limité.

18. Assemblage qui comprend :
(a) au moins deux éléments allongés (1, 3 ; 21, 23 ; 51, 52 ; 150, 151 ; 91, 93 ; 102, 106 ; 121, 123; 118, 119), positionnés le long l'un de l'autre, chacun ayant une partie de corps (7) et une partie d'extrémité (5 ; 29 ; 122, 124 ; 134, 135), les parties d'extrémité des éléments :
(i) étant capables d'être déployées vers l'extérieur, l'une à l'écart de l'autre, lorsqu'elles ne sont pas soumises à une contrainte transversale et présentant des surfaces de préhension ou coupantes à un objet devant être saisi ou coupé qui est placé entre elles en utilisation ; et
(ii) étant capables d'être déplacées vers l'intérieur l'une vers l'autre, pour saisir ou couper ledit objet ;
caractérisé en ce qu'une partie au moins de l'assemblage est appropriée pour être utilisée dans le dispositif selon l'une quelconque des revendications précédentes, et en ce qu'une partie d'au moins un des éléments est formée en un matériau qui présente une pseudo-élasticité lorsqu'il est déformé par une contrainte appliquée.
